(19) Europäisches Patentamt
European Patent Office
Office européen des brevets

(11) **EP 3 072 442 B1**

(12) **EUROPEAN PATENT SPECIFICATION**

(45) Date of publication and mention
of the grant of the patent:
**29.08.2018 Bulletin 2018/35**

(51) Int Cl.:
**A61B 5/00** *(2006.01)*      *A61B 3/032 (2006.01)*
**A61B 5/16** *(2006.01)*

(21) Application number: **16162196.6**

(22) Date of filing: **24.03.2016**

(54) **METHOD OF ASSESSING NEUROLOGICAL PHENOMENA OF HEMISPHERIC LATERALIZATION**

VERFAHREN ZUR UNTERSUCHUNG VON NEUROLOGISCHEN PHÄNOMENEN VON HEMISPHÄRISCHER LATERALISATION

PROCÉDÉ D'ÉVALUATION DES PHÉNOMÈNES NEUROLOGIQUES DE LATÉRALISATION HÉMISPHÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2015 IT MI20150434**

(43) Date of publication of application:
**28.09.2016 Bulletin 2016/39**

(73) Proprietor: **Universita' degli Studi di Bari
70121 Bari (IT)**

(72) Inventors:
• **MARTINO, Tommaso
70122 Bari (IT)**
• **DEFAZIO, Giovanni
70124 Bari (BA) (IT)**
• **QUARANTA, Nicola
70124 Bari (BA) (IT)**

• **LIVREA, Paolo
70124 Bari (BA) (IT)**

(74) Representative: **Valentini, Giuliano
Marietti Gislon e Trupiano S.r.l.
Via Larga 16
20122 Milano (IT)**

(56) References cited:
**US-A- 4 556 069**     **US-A1- 2003 109 799**

• **BELLIS ET AL: "Hemispheric lateralization of bilaterally presented homologous visual and auditory stimuli in normal adults, normal children, and children with central auditory dysfunction", BRAIN AND COGNITION, ACADEMIC PRESS, NEW YORK, NY, US, vol. 66, no. 3, 24 October 2007 (2007-10-24), pages 280-289, XP022503820, ISSN: 0278-2626**

**Description**

Field of the Invention

The present invention relates to a method for studying neurological phenomena of hemispheric lateralization, and in particular, the phenomena named as "neglect" and "pseudoneglect" of auditory and visual type.

The neglect neurological phenomenon occurs in subjects suffering from pathologies involving visuo-spatial and audio-spatial alterations, whereas the neurological pseudoneglect phenomenon normally occurs in healthy subjects and subjects suffering from pathologies not involving visuo-spatial or audio-spatial alterations.

Therefore the pseudoneglect degree does not belong to the pool of symptoms identifying the pathology. The method according to the present invention is not of diagnostic value, but has the aim of quantifying a normal phenomenon in order to provide quantitative results that can then be useful also in the study and assessment of the neglect phenomenon.

The invention provides carrying out a test series and acquiring the results from one or more subjects under consideration in order to allow a subsequent statistical analysis that allows assessing and quantifying the afore mentioned phenomenon.

State of the Art

The visuo-spatial neglect (visuo-spatial or hemispatial neglect) is a neurological syndrome characterized by the difficulty in reporting, responding and paying attention towards a stimulus submitted in the contralesional hemispace, defined in terms of retinotopic egocentric and allo centric coordinates.

The test more frequently used in order to determine the presence of a visuo-spatial hemineglect is the "Line Bisection Test" or "Line Bisection Task" (LBT), wherein the subject can be asked to trace a vertical dividing line in the center of a horizontal line or the subject can be asked to assess the position of the vertical dividing line on prebisected horizontal lines. Other types of tests used for this type of assessment are the Bell's Test, Double Letter Cancellation Task, Albert's Test, Barrage Test, Sentence Reading Test, Wundt-Jastrow Area Illusion Test (Pizzamiglio L., Judica A., Razzano C. & Zoccolotti P. - 1989 - "Toward a comprehensive diagnosis of visual-spatial disorders in unilateral brain damaged patients." Evaluacion Psicologica, 5, 199-218).

The pseudoneglect term, used for the first time in 1980, relates to the phenomenon whereby neurologically normal subjects show a systematic error in the Line Bisection Task (and like tests), which error generally leads to displacing to the left the perceived center with respect to the real one, when asked to bisect a horizontal line (Bowers D. & Heilman K. M. - 1980 - "Pseudoneglect. Effects of hemispace on a tactile line bisection task." Neuropsychologia, 18, 491-498).

There are several studies that analyzed this phenomenon, and not all the results agree with the afore mentioned observation about the error displacement to the left. There are in fact different studies wherein the pseudoneglect proved to be on the right.

Furthermore, the error extent (distance between perceived center and real center) is very variable among a variety of subjects. A meta-analysis considered 73 studies, for a total of 2191 neurologically healthy subjects. In such a study, 26 possible confounding factors have been weighted that can affect the results in the Line Bisection Task (Jewell G., McCourt M. E. & others - 2000 - "Pseudoneglect: A review and meta-analysis of performance factors in line bisection tasks." Neuropsychologia, 38, 93-110).

The prebisected lines used in the Line Bisection Task can be made of simple horizontal segments depicted on a screen or paper, or rectangular stimuli with polarity inversion (McCourt M. E. - 2001- "Performance consistency of normal observers in forced-choice tachistoscopic visual line bisection." Neuropsychologia, 39(10), 1065-1076), or also of trapezoidal lines with polarity inversion (McCourt M. E., Garlinghouse M. - 2000 - "Stimulus modulation of pseudoneglect: Influence of line geometry." Neuropsychologia, 38(4), 520-524).

Other studies further led to the adoption of vertical prebisected lines in order to assess the so called "altitudinal (or vertical) pseudoneglect" phenomenon, in addition to that so far stated and related to the "horizontal (or azimuthal) pseudoneglect" phenomenon (Suavansri K., Falchook A. D., Williamson J. B., & Heilman K. M. - 2012 - "Right up there: Hemispatial and hand asymmetries of altitudinal pseudoneglect." Brain and cognition, 79(3), 216-220).

Previous studies on pseudoneglect often used the Line Bisection Task as visual lateralization index. As already noted previously, this test can be carried out in two different ways: the subject can be asked to bisect a horizontal line (in different ways: by means of a laser pointer, by using a pen, etc.); alternatively, the subject can be asked to judge whether a pre-bisected stimulus has the two equivalent halves or if they are different (see for example: Rueckert L., Deravanesian A., Baboorian D., Lacalamita A., & Repplinger M. - 2002 - "Pseudoneglect and the cross-over effect." Neuropsychologia, 40(2), 162-173). In both cases, it is possible to incur different "biases" (also defined as "stretches" or "distortions").

In case the subject is asked to manually bisect a submitted line, the subject can designate the subjective center as lateralized with respect to the real (objective) center, not for a center perception bias, rather for a motor bias. The perception of a line center can be correct, but the "perception - movement" translation can be somehow altered (see for example the discussion in: Chen P., Goedert K. M., Murray E., Kelly K., Ahmeti S. & Barrett A. M. - 2011 - "Spatial bias and right hemisphere function: Sex-specific changes with aging." Journal of the International Neuropsychological Society, 17, 455.).

On the contrary, in studies using pre-bisected lines ("landmark stimuli"), the task always requested to judge whether the center marked on the line was positioned on the right or on the left with respect to the perceived center, thus using a protocol called "two alternative forced choice judgment" (see for example: Sosa Y., Clarke A. M., & McCourt M. E. - 2011- "Hemifield asymmetry in the potency of exogenous auditory and visual cues." Vision research, 51(11), 1207-1215). Also this kind of test execution can represent a stretch. For laterally pre-bisected lines but very close to the real line center, the subject is forced to select between "right" and "left", despite he/she can believe the line correctly pre-bisected. The selection appears therefore totally subjective and can not be related to what the subject really perceives.

Concerning, on the contrary, the lateralization ability of auditory stimuli, despite there are a number of studies on this topic in literature, none of them developed a method validated for the research of the auditory neglect/pseudoneglect phenomena.

Similarly to what already observed for the visual stimuli, experiments heretofore adopted of auditory lateralization ask for a judgment between right and left, once again forcing the subject to select between two responses that can both appear, in fact, "wrong" to the subject, since he/she perceives the sound as central.

The document "Hemispheric lateralization of bilaterally presented homologous visual and auditory stimuli in normal adults, normal children, and children with central auditory dysfunction" - Brain and cognition, 66(3), 280-289; Bellis, T. J., Billiet, C., & Ross, J. (2008), aims to demonstrate a hemispherical lateralization in the field of the functional asymmetry of the areas devoted to the language perception and processing (decoding). Such a research topic often uses *dichotic listening* experiments, during which two different auditory stimuli are sent simultaneously to the two ears, subsequently asking the subject which of the two stimuli was perceived. Such experiments use verbal stimuli, generally simple syllables (*consonant-vowel syllables*), such as for example ba/ga/pa, different for the two acoustic channels. For example, the "ba" syllable is made heard on the right and simultaneously the "ga" syllable on the left, and it is proven that an advantage on the right ear (*right-ear advantage,* REA) can be noted and, thus, a dominance of the contralateral (left) hemisphere in the decoding of verbal acoustic stimuli. The REA is present at all ages, both in males and females, in left-handed and right-handed subjects.

By considering that acoustic information projected up to the primary auditory cortex are mainly, but not exclusively, contralateralized, a possible REA explanation is the dominance of the left hemisphere for the language perception and decoding, this hemisphere being the seat of the Broca and Wernicke cerebral areas, responsible for the language perception and production.

While the dichotic listening of syllables or acoustic information relating to other language mainly activates the left hemisphere, the listening of non-verbal sounds (for example musical instruments) mainly activates the right hemisphere, thereby configuring the *Left-Ear Advantage* (LEA) phenomenon. The LEA in general occurs for non-verbal auditory cues such as environmental sounds, musical instruments or non-verbal human sounds. The set of these data thereby configures the so called *auditory laterality effect,* related to verbal or non-verbal dichotically submitted stimuli.

However, the state of the scientific art provides that the *dichotic listening* used in experiments and paradigms (*dichotic listening paradigm*) published in literature is made of different stimuli, which are simultaneously submitted to the two ears. Specifically, by analyzing the procedure of the document by Bellis et al., it can be deduced that *"...pairs of monosyllabic digits from 1 to 10 ... were delivered simultaneously to each of the two ears, with each ear receiving a different digit pair...".* Therefore, for example, the number "one" is made heard to the right ear and number "two" to the left ear. The subsequent analysis of the responses given by the participants allows inferentially obtaining data about the advantage of the right (REA) or left (LEA) ear in acknowledging (decoding) the simultaneously-submitted verbal acoustic stimuli.

Similarly, the visual variation of the auditory task used in the document by Bellis et al. provides that *"...a black cross ... served as a central fixation marker. One pair of digit (one digit to the right and left of the central fixation marker) was presented...".* The subsequent analysis of the responses given by the participants allows inferentially obtaining data about the number acknowledgment (decoding) ability in the right (*right visual field advantage,* RVFA) and left (*left visual field advantage,* LVFA) hemifields, respectively.

US-A-4556069 describes a method for diagnosing dyslexia, consisting in the submission of two sound stimuli by means of headphones, that are modified in the ITD parameter during the subsequent tests to the extent that the sound stimulus is perceived as localized in the center of the subject concerned.

In greater detail, this document provides for a train of sound stimuli (lasting 150 microseconds, with administration frequency of 7.6 Hz) being submitted via headphones to the subject, who will specify the subjective origin source of the sound stimulus. Through dedicated push buttons the operator will be able to modify the ITD parameter by 8 microseconds at a time (until a maximum of 8 times), thus modifying the subjective perception of the origin of the following stimuli, to the extent where the subject will perceive the sound clicks as originating from their own center. The method for assessing the subjective perception of the audio-spatial center is therefore only based on the change of the ITD parameter. Furthermore, this document does not differentiate the study of the auditory right hemifield and left auditory hemifield.

Object of the present invention is to provide a method for studying neurological phenomena of hemispheric lateralization, that allows avoiding the bias of interpretation by the subject.

Another object of the present invention is to provide a method of the afore mentioned type that allows detecting the presence of the pseudoneglect phenomenon and providing a set of variables that allow quantifying the extent with a standardized approach.

A further object of the present invention is to provide a method that further allows detecting the presence also of the only auditory pseudoneglect phenomenon in normal subjects.

Summary of the invention

These and other objects are reached by a method for assessing neurological phenomena of hemispheric lateralization, the method comprising the steps of:

a) providing a programmed test apparatus to submit a test series to a subject and acquire the respective responses from the subject, the test apparatus comprising at least one computer, at least one monitor for displaying images, at least one audio reproducing device and means for selecting the test responses;

b) submitting to the subject at least one visual stimulus selected randomly from a set of visual stimuli made of prebisected lines, or rectangular stimuli, trapezoidal stimuli or triangular stimuli with polarity inversion, in variable position, and acquiring from the subject a response selected from a set of responses to visual stimuli;

c) submitting to the subject at least one auditory stimulus selected randomly from a set of sinusoidal or complex auditory stimuli, identical to each other in the binaural presentation, with changes in the ILD and/or ITD and/or IPD parameters, and acquiring from the subjects a response selected from a set of responses to the auditory stimuli;

d) repeating the steps b) and c) in random order for a fixed number of times;

e) calculating a numerical value representative of the neurological phenomenon of visuo-spatial and audio-spatial attention lateralization on the basis of responses given by the subject to different visual and auditory stimuli.

With respect to the *dichotic listening* paradigms for the study of the REA/LEA in the decoding of verbal cues, according to which different verbal or non-verbal sound stimuli are used between the two right/left sound channels, the inventive method provides for the use of the same sound (for example pure sinusoidal) as acoustic stimulus.

Furthermore, in the *dichotic listening* paradigms, the verbal or non-verbal sounds are not modified in the ITD (*interaural time difference*), ILD (*interaural level difference*), IPD (*interaural phase difference*) parameters, which is on the contrary an essential requirement in the method according to the invention.

Similarly, in the visual counterpart, the study of the document by Bellis et al. provides for two different visual stimuli being submitted in the two visual hemifields. On the contrary, in the method according to the invention the visual stimulus is similar in all the stimuli (e.g. a line bisected by a transector) with change of the transector localization parameter expressed in degrees/radiants with respect to the observer.

In both the spheres (visual and auditory) according to the document by Bellis et al., the aim is to demonstrate a hemispheric dominance in decoding the stimuli, whereas the method according to the present invention aims to identify the (egocentric) coordinates of the visuo-spatial and audio-spatial perception.

Finally, in the paradigms described by the document by Bellis et al., as well as other literature papers, the stimuli submission does not fix, a priori, a visual/auditory study hemifield, but mixes stimuli that can be variously perceived from the right or the left, without taking into account the variable of perception from a point centered with respect to the observer. Conversely, this last aspect falls in the scope of the present invention. The quantification of hemispheric lateralization phenomena, and in particular of the visual and auditory pseudoneglect phenomenon, allows obtaining threshold values (cutoff) useful for comparing normal subjects with subjects suffering from neurological pathologies. In this way, the present study can be considered as a preliminary approach for subsequent studies in normal populations, but also has possible usage in understanding cerebral mechanisms (if it is carried out, for example, while acquiring a cerebral signal, such as the electroencephalogram or functional magnetic resonance) and neurological symptoms.

The set of visual stimuli can include, for example, horizontal lines bisected in the center, on the right of the center and on the left of the center, or vertical lines bisected in the center, beneath the center and over the center, or still rectangular, trapezoidal and triangular stimuli with polarity inversion in the center, on the right of the center and on the left of the center.

The choice of using pre-bisected lines or images allows avoiding the motor bias (cross-over effect) that could affect the subject response.

The set of auditory stimuli includes balanced simple tones, simple tones lateralized on the right and simple tones lateralized on the left. Each one of the auditory stimuli submitted to the subject can also be preceded by a distractor stimulus having prefixed duration. The method according to the present invention provides the possibility of assessing the (visual and auditory) stimuli as central ones, so as to avoid the bias forcing the response. In practice, the set of responses to the visual stimuli includes the assessment of center bisection of horizontal or vertical lines and center polarity inversion. In the same way, the set of responses to the auditory stimuli includes the assessment of balanced simple tones. In the method according to the invention, the same number of visual stimuli is submitted to each subject per each of the two hemifields, and the same number of auditory stimuli per each of the two hemifields. This allows investigating the ability of perceiving visual and auditory stimuli as lateralized, separately per each of the two visual hemifields and per each of the two auditory fields, thus avoiding any bias of forced response. The two afore described response biases (response forcing and erroneous perception-motion translation) are avoided.

The method further includes the separate calculus of the percentages of correct responses per each of the two visual hemifields and per each of the two auditory hemifields, and comprises the possibility of obtaining a comparison between the performances obtained in the two hemifields.

Brief Description of the Drawings

Further characteristics and advantages of the present invention will be more evident from the following description made by way of example and referring to the attached drawings, in which:

- Figure 1 is a flowchart showing some steps of the method according to the present invention;
- Figures 2A-2C show some examples of pre-bisected lines used in the method according to the present invention;
- Figures 3A-3C show some examples of rectangular stimuli with polarity inversion that are used in the method according to the present invention;
- Figures 4A-4C show some examples of triangular stimuli with polarity inversion that are used in the method according to the present invention;
- Figure 5 is a plot depicting the results of the visual tests; and
- Figure 6 is a plot depicting the results of the auditory tests.

Detailed description

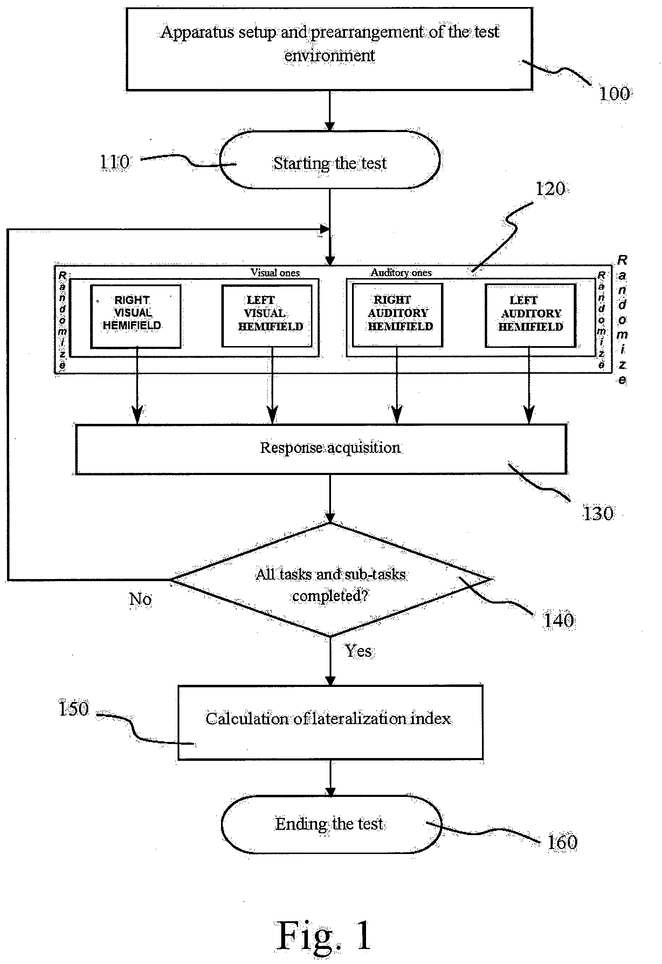

Following the flowchart of Figure 1, the method initially provides a step 100 during which the apparatus is prepared, for example a personal computer, that has to be used during the test, and the environment in which the various tests (in the following also defined as tasks and sub-tasks) will be carried out is prearranged. In this step, in addition to fixing the number and typology of the sub-tasks the subject has to be subjected to, the brightness of the environment, the environmental noise, the distance of the subject from the display on which the visual stimuli appear, as well as the volume and the position of the headphones through which the auditory stimuli are submitted, are assessed, for example.

At the test start (step 110) at least one screen of "instructions" is shown on the display (for example to specify what the experiment is, which keys to press for answering, etc.). Alternatively, an operator explains the way to proceed.

After starting the test, various visual and auditory stimuli are submitted to the subject. This step is depicted by the step 120 in which the visual and auditory stimuli are submitted to the subject with random sequence and modality; "randomize" terms shown on the diagram denote that the various stimuli associated with each sub-task are submitted with random sequence. In particular, the stimuli submission is preferably "randomized" with the following order:

- randomization 1: the submission program randomly selects whether starting from the visual task or else the auditory task. Only at the end of one of the two tasks, the subsequent one follows;
- randomization 2: once the task has been selected, the submission program randomly selects the sub-task, by selecting whether starting from the set of central/right lateralized stimuli, or else of central/left lateralized stimuli. Only at the end of one of the two sub-tasks, the subsequent one follows;
- randomization 3: once the sub-task has been selected, the submission program randomly selects the submission order of the various stimuli.

The visual stimuli to be judged are displayed on the screen of the computer running the test program, whereas the auditory stimuli are submitted via headphones. Both the visual and auditory stimuli can be preceded by a "distractor" stimulus and by a short pause, in order to prevent any form of comparison of the current stimulus with the previous stimulus, on the contrary allowing an independent judgment for each stimulus. For this purpose, in case of visual stimuli, these will be shown on the screen, by varying their primary position in the four directions (stimulus displaced upwardly, followed by stimulus displaced downwardly/to the left/to the right) by few degrees: these two precautions (presence of the distractor stimulus, displacement of the primary position) serve to prevent the previous stimulus from affecting the response of the current stimulus. At step 130 the response to the visual/auditory stimulus submitted in a sub-task and other possible parameters, for example the subject response time to the submitted stimulus, are acquired.

At the decision block 140, it is checked whether all the previously planned sub-tasks have been submitted to the subject.

If not, it goes back to step 120 in order to submit a new stimulus to the subject and this is repeated until reaching the established number of sub-tasks. In this case it is important to note that, in each test a subject is submitted to, the number of visual stimuli related to the right hemifield must be the same as the number of visual stimuli for the left hemifield, and the same applies as regard to the auditory stimuli.

If so, the block 150 follows, in which the lateralization indices per each hemifield are calculated. In this step, in addition to storing the obtained results, additional result analyses can be carried out, for example by displaying characteristic curves based on test results or by comparing the same with statistical data previously stored for a given person population. The test can thus be considered concluded (block 160).

By way of example and without limitation, examples related to some particular aspects of the method according to the present invention are reported below.

Example 1: Auditory stimuli and characteristics thereof

The auditory stimuli used in the method according to the present invention are preventively generated by means of convenient software functions and stored on a storage medium readable by the computer for carrying out the test. The set of the so generated auditory stimuli includes, for example, balanced simple tones, simple tones lateralized on the right and simple tones lateralized on the left with respect to the subject listening to the same via headphones.

The sounds constituting the auditory stimuli to be submitted to the subject are generated at least depending on the following parameters:

- sound frequency, expressed in Hz;
- sound duration in seconds;
- sampling rate, expressed in Hz (for example 48000 Hz)
- sound quality, expressed in bit (for example 16 bit)
- volume of left channel (expressed for example as percent value between 0 and 100);
- volume of right channel (expressed for example as percent value between 0 and 100);
- origin angle, expressed in degrees or radiants.

In order to obtain a lateralized auditory stimulus, an origin angle different from zero (by modifying for this purpose the Interaural Time Difference parameter - meant as initial difference of sound in the two headphone audio channels - and accordingly the Interaural Phase Difference parameter) can be specified, for example, or else the volume percent of the right channel can be varied with respect to the left channel (i.e. by modifying the Interaural Level Difference parameter - meant as volume percent difference between the two headphone audio channels), or even by using a combination of these variable parameters. The set of auditory stimuli also comprises balanced simple tones, i.e. sounds having a null origin angle and identical percents for the volumes of the right and left channels.

For example, if $\theta$ is the origin angle expressed in degrees, the synthesized sound will have an Interaural Time Difference (ITD) calculated on the basis of the following Woodworth formula:

$$ITD = (0.09 \cdot (\theta + \sin\theta))/344$$

in which 0.09 expresses, in meters, half the standard distance between the two ears, the sound origin angle $\theta$ is expressed in radiants, and 344 is the sound speed expressed in m/s.

In addition, or alternatively, the sound percent volume in the two audio channels can be separately modulated, by specifying a different percent value for the two channels. This way, in addition to the above formulated ITD, also the Interaural Level Difference (ILD) is modulated.

The set of auditory stimuli further includes sounds such as those so far defined that can be preceded by a distractor sound. In this case, in addition to the afore mentioned parameters, the distractor sound duration expressed in seconds (for example 1.5 seconds) and the pause duration still expressed in seconds, between the distractor sound and the actual sound stimulus (for example 0.5 seconds) can be specified, for example. Also the waveform of the distractor sound can be set so as to be different from the lateralized or balanced sound submitted to the subject immediately after.

Example 2: Visual stimuli and characteristics thereof

Some examples of visual stimuli, prearranged for being carried out in the method according to the invention, are shown below.

Also in this case, the images constituting the visual stimuli are preventively generated by means of convenient software functions and stored on a storage medium readable by the computer with which the test is carried out. The set of so generated visual stimuli includes for example prebisected lines, rectangular stimuli and triangular stimuli that are displayed to the subject on the monitor connected to the computer running the test program.

Example 2.1: Prebisected lines

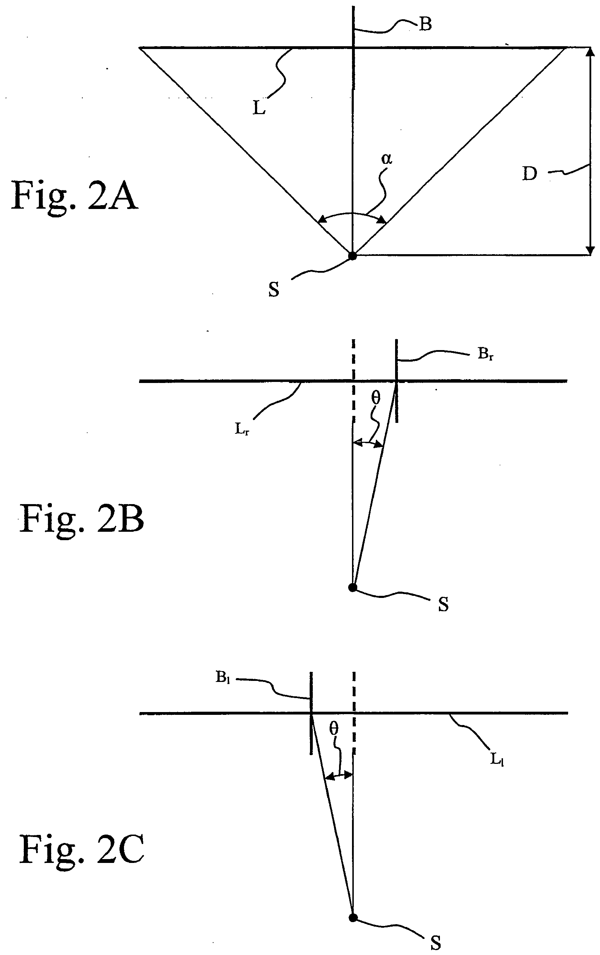

Some examples of prebisected horizontal lines are depicted in Figures 2A-2C to point out the parameters characterizing the same.

In Figure 2A, a horizontal line L is shown as prebisected by a vertical transector segment B placed exactly in the center of the length of the line L. With respect to the point S, which identifies the center of the observer eyes, the distance D of the line L from the observer in the point S is expressed in centimeters, for example, and the line total length is expressed by an angle $\alpha$ denoting the subtended visual angle between the center S of the observer eyes and the ends of the horizontal line L, expressed for example in degrees or radiants.

Other characteristic parameters of the lines L and B can include the thickness of the horizontal line, expressed for example in degrees or radiants as a function of the visual angle subtended between the center S of the observer eyes and the height of the horizontal line, the graphic resolution of the lines, expressed for example in dpi (dot per inch) or ppi (pixel per inch), the color of the horizontal line L and the color of the transector segment B. The graphic resolution of the lines is preferably suited to the resolution of the screen on which the lines are shown.

In Figure 2B a line $L_r$ bisected by a transector segment $B_r$ displaced to the right with respect to the center (depicted by a dotted segment) of the line $L_r$, is shown. In addition to the parameters already shown for the line L of Figure 2A, also the angle $\theta$ defining the position in degrees of the transector segment $B_r$ is specified as the visual angle subtended between the center S of the observer eyes and the position of the transector segment $B_r$ with respect to the center of the line $L_r$.

Conversely in Figure 2C a line $L_1$ bisected by a transector segment $B_1$ displaced to the left with respect to the center (depicted by a dotted segment) of the line $L_1$, is shown. Also in this case, the position of the transector segment $B_1$ is defined by the visual angle $\theta$.

If the angle $\theta$ is null, the transector segment is positioned exactly in the center of the line (Figure 2A) whereas, for positive and negative values of the angle $\theta$, the transector segment will be on the right or on the left with respect to the center (Figure 2B or 2C). Similar parameters can also be used even if vertical prebisected lines rather than horizontal lines or in combination with the latter will be submitted to the subject. Therefore, the vertical lines can be bisected in the center, beneath the center or over the center.

In practice, in order to obtain for example the vertical prebisected lines, the same software functions generating the horizontal lines can be used and the resulting image can be saved in a graphic file (for example of the png, jpg or the like) after the same have been rotated by 90° with a common image processing program. Such stimuli will assess the presence of the "vertical pseudoneglect" phenomenon.

Example 2.2: Rectangular stimuli with polarity inversion

The set of visual stimuli also includes the rectangular stimuli having dimensions specified upon the visual angle, in which the color (or polarity) inversion occurs exactly in the center, otherwise on the left or on the right of the real center with respect to an angle specified too.

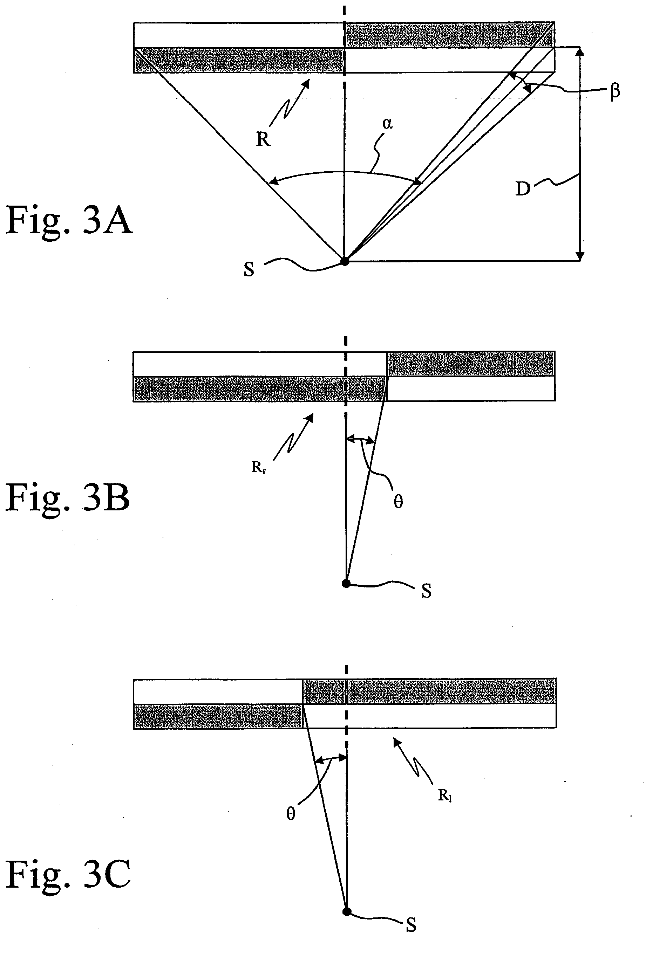

In Figure 3A the image of a rectangle R is shown, in which the polarity inversion is arranged exactly in the center. Similarly to what already depicted for the prebisected lines, the point S denotes the center of the observer eyes, the distance D of the rectangle R from the observer is expressed for example in centimeters, and the total length of the rectangle R is defined by the angle $\alpha$ expressed in degrees or radiants, i.e. the visual angle subtended between the center S of the observer eyes and the ends of the rectangle R. The height (or thickness) of the rectangle R is rather defined by the angle $\beta$ expressed in degrees or radiants, i.e. the visual angle subtended between the center S of the observer eyes and the rectangle height.

Other characteristic parameters of the rectangle R can include the graphic resolution expressed for example in dpi (dot per inch) or ppi (pixel per inch) and the colors of the areas for the two different polarities.

A rectangle $R_r$ in which the polarity inversion is displaced to the right with respect to the geometrical center (intersected by a dotted line) of the rectangle $R_r$, is depicted in Figure 3B. In addition to the parameters already shown for the rectangle R of Figure 3A, also the angle $\theta$ defining the position in degrees of the polarity inversion point is specified as the visual angle subtended between the center S of the observer eyes and the position of the inversion point.

Conversely, in Figure 3C a rectangle $R_1$ is depicted in which the polarity inversion is displaced to the left with respect to the center of the rectangle $R_1$. Also in this case, the position of the inversion point is defined by the visual angle $\theta$.

In practice, as already defined for the prebisected lines of the Example 2.1, if the angle $\theta$ is null, the inversion point is positioned exactly in the center of the rectangle (Figure 3A) whereas, for positive and negative values of the angle $\theta$, the transector segment will be on the right or on the left with respect to the center (Figure 3B or 3C). Also for this type of stimuli, the orientation of the larger dimension of the rectangle can be adapted, in order to assess the vertical pseudoneglect.

Example 2.3: Triangular stimuli with polarity inversion

The set of visual stimuli can also include triangular stimuli having dimensions specified upon the visual angle, in which the color (or polarity) inversion occurs exactly in the center, otherwise on the left or on the right of the real center with respect to an angle specified too.

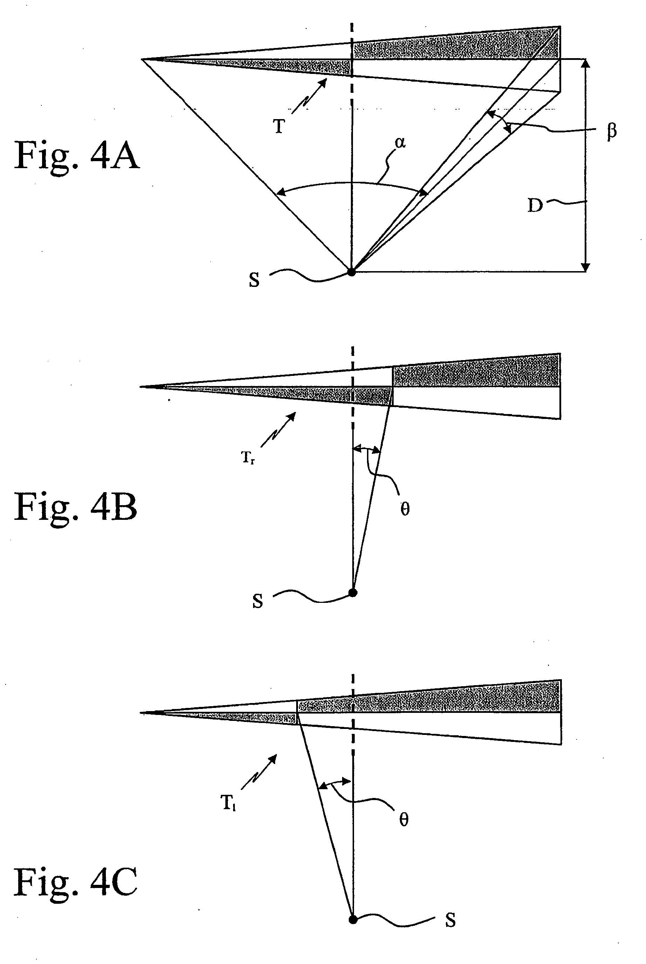

In Figure 4A the image of a triangle T is shown, in which the polarity inversion is arranged exactly in the center. Similarly to what already depicted in the Example 2.2, the point S denotes the center of the observer eyes, the distance D of the triangle T from the observer is expressed for example in centimeters, and the total length of the triangle T is defined by the angle $\alpha$ expressed in degrees or radiants, i.e. the visual angle subtended between the center S of the observer eyes and the ends of the triangle T. The maximum height dimension of the triangle T, measured at the geometric base, is on the contrary defined by the angle $\beta$ expressed in degrees or radiants, i.e. the visual angle subtended between the center S of the observer eyes and the triangle base.

Other characteristic parameters of the triangle T can include the graphic resolution expressed for example in dpi (dot per inch) or ppi (pixel per inch) and the colors of the areas for the two different polarities.

A triangle $T_r$ in which the polarity inversion is displaced to the right with respect to the center (intersected by a dotted line) of the triangle $T_r$, is depicted in Figure 4B. In addition to the parameters already shown for the triangle T of Figure 4A, also the angle $\theta$ defining the position in degrees of the polarity inversion point is specified as the visual angle subtended between the center S of the observer eyes and the position of the inversion point.

Conversely, in Figure 4C a triangle $T_1$ is depicted in which the polarity inversion is displaced to the left with respect to the center of the triangle $T_1$. Also in this case, the position of the inversion point is defined by the visual angle $\theta$.

In practice, as already defined for the rectangular tachistoscopic stimuli of the example 2.2, if the angle $\theta$ is null, the inversion point is positioned exactly in the center of the triangle, i.e. exactly half the geometric height (Figure 4A) whereas, for positive and negative values of the angle $\theta$, the transector segment will be on the right or on the left with respect to the center (Figure 4B or 4C).

The same considerations herein reported for the triangular stimuli also apply for other shapes the stimuli can take in general, for example for trapezoidal and like stimuli.

Example 3: Graphic representation of the results

The program used to manage the test further includes some functions for graphically and quantitatively examining (by means of the extraction of some parameters described below) the performance of a single subject in the two visual tasks and/or the two auditory tasks related to the right and left visual hemifields.

A possible graphic representation of the performance of the single subject can be obtained by using techniques of known type, such as the generalized linear regression (GLM) with categorical response, such as the logistic regression (logit model) or variations thereof (probit model). As regards the population, the performance of a group of subjects can be graphically depicted (and subsequently analyzed) by using techniques such as GLMM (generalized linear mixed model), as described elsewhere (Moscatelli, A., Mezzetti, M., & Lacquaniti, F. (2012). Modeling psychophysical data at the population-level: The generalized linear mixed model. Journal of vision, 12(11), 26).

Example 3.1: Results of the visual tasks

Let's suppose a subject performs the two visual tasks, respectively in the visual right and left hemifields, assessing the position of a transector segment of a prebisected line; the responses available to the subject are three, i.e. that one of bisection in the center of the line, and the bisection ones on the right or on the left with respect to the line center. The collected data are shown in Table 1 reported below.

Table 1

| cnt | Right | | Left | |
|---|---|---|---|---|
| | Number of correct responses | Number of erroneous responses | Number of correct responses | Number of erroneous responses |
| 0 | 0 | 10 | 0 | 10 |
| 0.1 | 2 | 8 | 0 | 10 |
| 0.2 | 2 | 8 | 0 | 10 |
| 0.3 | 3 | 7 | 0 | 10 |
| 0.4 | 2 | 8 | 0 | 10 |
| 0.5 | 4 | 6 | 0 | 10 |
| 0.6 | 8 | 2 | 1 | 9 |
| 0.7 | 10 | 0 | 4 | 6 |
| 0.8 | 8 | 2 | 5 | 5 |
| 0.9 | 10 | 0 | 7 | 3 |
| 1 | 9 | 1 | 8 | 2 |
| 1.1 | 10 | 0 | 7 | 3 |
| 1.2 | 10 | 0 | 10 | 0 |
| 1.3 | 10 | 0 | 10 | 0 |
| 1.4 | 10 | 0 | 10 | 0 |

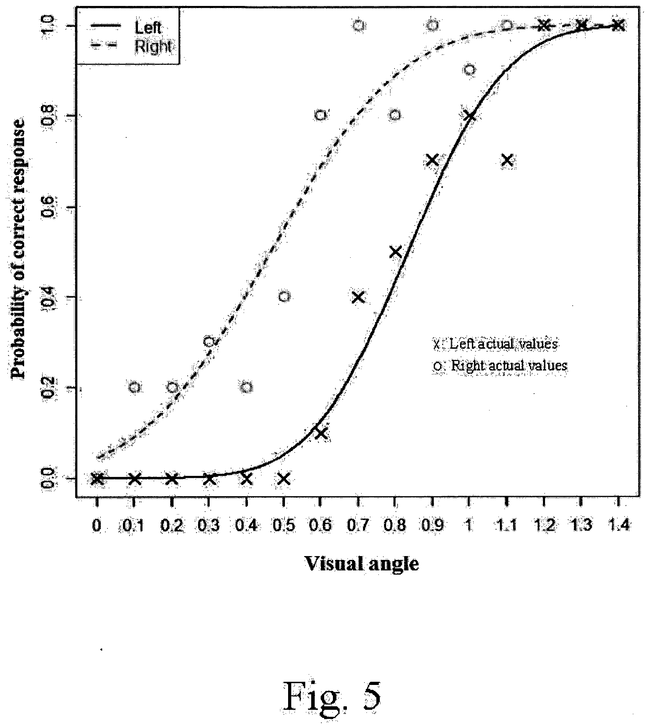

The values of visual angle, expressed in degrees, defining the position of the transector segment in each single sub-task (angle $\theta$ in Example 2.1) are specified in "cnt" column of Table 1. Each visual angle has been shown 10 times. The employed function allows graphically depicting the curves of Figure 5 by approximating the curve trends to the actual results by means of algorithms or techniques of known type.

The quantitative analysis of the results shown in Table 1 and of the plot obtained in Figure 5 can include, for example, functions aiming to calculate a measure of the area subtended to each of the two curves of Figure 5, as well as the values, expressed in degrees, corresponding to a given percent probability of correct response and extrapolated by each of the two curves.

Example 3.2: Results of the auditory tasks

The same function as the Example 3.1 can be used in order to analyze the performance of a subject in the auditory task. Let's suppose a subject performs the two auditory tasks as those characterized in the Example 1, i.e. by asking the subject to judge the origin of each sound, respectively in the auditory right and left hemifields; the responses available to the subject are three, i.e. that one of a balanced sound, and those of sounds coming from the right or the left with respect to the subject. The results are shown in Table 2 reported below.

Table 2

| cnt | Right | | Left | |
|---|---|---|---|---|
| | Number of correct responses | Number of erroneous responses | Number of correct responses | Number of erroneous responses |
| 0 | 1 | 9 | 0 | 10 |
| 82 | 3 | 7 | 0 | 10 |
| 163 | 6 | 4 | 2 | 8 |
| 242 | 10 | 0 | 4 | 6 |
| 318 | 9 | 1 | 6 | 4 |

(continued)

| cnt | Right | | Left | |
|---|---|---|---|---|
| | Number of correct responses | Number of erroneous responses | Number of correct responses | Number of erroneous responses |
| 390 | 10 | 0 | 9 | 1 |
| 458 | 10 | 0 | 10 | 0 |
| 521 | 10 | 0 | 10 | 0 |
| 578 | 10 | 0 | 10 | 0 |
| 628 | 10 | 0 | 10 | 0 |
| 673 | 10 | 0 | 10 | 0 |

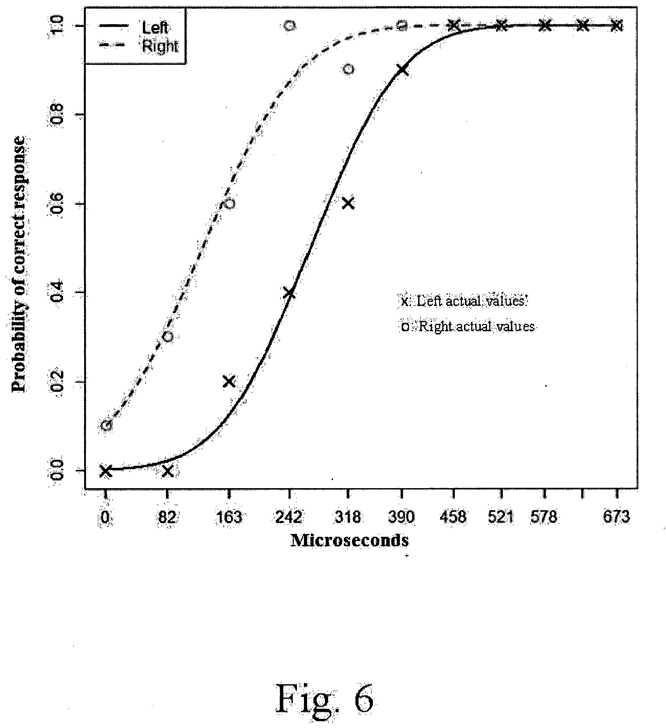

The lateralization values used for the auditory stimuli, in particular the sound duration expressed in microseconds, are shown in the "cnt" column of the table above. Each lateralized sound stimulus has been submitted 10 times. The employed function allows graphically depicting the curves of Figure 6 by approximating the curve trends to the actual results by means of algorithms or techniques of known type.

The quantitative analysis of the results shown in Table 2 and of the plot obtained in Figure 6 can include, for example, functions aiming to calculate a measure of the area subtended to each of the two curves of Figure 6, as well as the values, expressed in microseconds, corresponding to a given percent probability of correct response and extrapolated by each of the two curves.

Other parameters that can be extrapolated by applying the suitable statistical techniques of known type and mentioned above (GLM, logistic regression, probit model, GLMM), comprise for example: (1) the "point of subjective equality" (PSE), corresponding to the value for which the percent of correct response is equal to 50% (p50); (2) the "difference limen", defined as the difference between the value to which 75% of correct responses correspond (p75) and the value to which 25% of correct responses correspond (p25); (3) the "Weber ratio" (WR), defined by the following formula:

$$WR = (p75\text{-}p25) / (2*p50)$$

Example 4: Calculation of the lateralization index

In literature there is not unanimous agreement on calculating a lateralization index LI in psychometric tasks of visual or auditory lateralization tests.

Some formulas are proposed below for calculating possible LI values, that allow synthetically describing whether the performance in the visual/auditory right hemifield is better or worse than the performance in the visual/auditory left hemifield, thus allowing quantifying the extent of the lateralization phenomenon.

For example, a significant value of a possible lateralization index can be calculated as the difference between the equivalent percent probabilities of correct response in each of the visual or auditory tasks for the right hemifield and the left hemifield. A positive or negative value resulting from this difference can be indicative of a better performance obtained for the right hemifield with respect to the left hemifield, or vice versa. Another possible value can be obtained for example also by the difference between the area subtended to the two curves depicted in Figure 5 (visual task), as well as between the area subtended to the two curves of Figure 6 (auditory task).

Other possible values for the lateralization index LI can be obtained on the basis of the number of correct responses given by the subject in a visual task or an auditory task. A possible formula is the following:

$$LI = (NR\text{-}NL) / 0.5(NR\text{+}NL)$$

wherein NR is the total number of correct responses in the right auditory or visual task (R = right), and NL is the total number of correct responses in the left auditory or visual task (L = left).

Another possible formula is the following:

$$LI = NR/tot - NL/tot$$

wherein "tot" is the total number of responses given by the subject in each task, whereas NR and NL have the same meaning as the previous formula. The formula thus depicts a probability difference.

Another possible applicable formula is the following:

$$LI = rPSE - lPSE$$

wherein rPSE is the "point of subjective equality" in the right auditory or visual sub-task, and the lPSE is the "point of subjective equality" in the corresponding left sub-task.

On the basis of these formulas, when LI > 0 it means that the performances in the right hemifield are better than the performances in the left hemifield.

Various changes can be made with respect to the embodiments herein depicted, by way of example only without departing from the scope of the present invention. For example, even if the method has been heretofore mainly described for the study of the neurological "pseudoneglect" phenomenon, the same principles can also be applied to the study of the neurological "neglect" phenomenon.

Another variation example is the following. In the submission of the visual stimuli, one can select whether to use a neutral background (for example the white color) or a differently depicted background (for example two equal or different symbols or images being at one or both the sides of the visual stimulus), in order to assess the extent of the pseudoneglect phenomenon in baseline conditions (neutral background) and in presence of other elements in the subject's perceptive space. Similarly, for the same purpose during the submission of auditory stimuli, other variously submitted auditory stimuli (for example a music, a lateralized interfering tone, etc.) can be associated.

**Claims**

1. A method of assessing neurological phenomena of hemispheric lateralization, the method comprising the steps of:

   a) providing a programmed test apparatus to submit a test series to a subject and acquire the respective responses from the subject, the test apparatus comprising at least one computer, at least one monitor for displaying images, at least one audio reproducing device and means for selecting the test responses;
   b) submitting to the subject at least one visual stimulus selected randomly from a set of visual stimuli made of prebisected lines, or rectangular stimuli, trapezoidal stimuli or triangular stimuli with polarity inversion, in variable position, and acquiring from the subject a response selected from a set of responses to visual stimuli;
   c) submitting to the subject at least one auditory stimulus selected randomly from a set of sinusoidal or complex auditory stimuli, identical to each other in the binaural presentation, with changes in the ILD and/or ITD and/or IPD parameters, and acquiring from the subject a response selected from a set of responses to the auditory stimuli;
   d) repeating the steps b) and c) in random order for a fixed number of times;
   e) calculating a numerical value representative of the neurological phenomenon of visuo-spatial and audio-spatial attention lateralization on the basis of responses given by the subject to different visual and auditory stimuli.

2. The method according to claim 1, wherein the set of said visual stimuli includes horizontal lines bisected in the center, on the right of the center and on the left of the center.

3. The method according to claim 1, wherein the set of said visual stimuli includes vertical lines bisected in the center, beneath the center and over the center.

4. The method according to claim 1, wherein said set of said visual stimuli used in step b) of claim 1 includes rectangular, trapezoidal and triangular stimuli with polarity inversion in the center, on the right of the center and on the left of the center.

5. The method according to claim 1, wherein said set of said auditory stimuli used in step c) of claim 1 includes balanced simple tones, simple tones lateralized on the right and simple tones lateralized on the left.

**6.** The method according to claim 1, wherein said set of responses to said visual stimuli obtained in step b) of claim 1 includes the assessment of center bisection of horizontal or vertical lines and stimuli with polarity inversion.

**7.** The method according to claim 1, wherein the set of responses to said auditory stimuli includes the assessment of balanced simple tones.

**8.** The method according to claim 1, wherein at least one auditory stimulus submitted in said step c) is preceded by a distractor stimulus.

**9.** The method according to claim 1, wherein the same number of visual stimuli is submitted to each subject per each of the two hemifields, and the same number of auditory stimuli is submitted per each of the two hemifields.

**10.** The method according to claim 1, wherein said step e) includes calculating individually the percentages of correct responses per each of the two visual hemifields and per each of the two auditory hemifields.

**11.** A computer program product, comprising a data medium in which executable line codes, which are realized in machine readable form, are stored for carrying out a method according to claims 1 to 10, when run in a programmed test apparatus to submit a test series to a subject and acquire the respective response from the subject, the test apparatus comprising ate least a computer, at least one monitor for displaying images, at least one audio reproducing device and means for selecting test responses.

**Patentansprüche**

**1.** Verfahren zum Bewerten von neurologischen Phänomenen der hemisphärischen Lateralisierung, wobei das Verfahren die folgenden Schritte umfasst:

a) Bereitstellen einer programmierten Testvorrichtung zum Übermitteln einer Testreihe an ein Subjekt und zum Erfassen der jeweiligen Antworten von dem Subjekt, wobei die Testvorrichtung mindestens einen Computer, mindestens einen Monitor zum Anzeigen von Bildern, mindestens ein Audiowiedergabegerät und Mittel zum Auswählen der Testantworten umfasst;
b) Unterwerfen des Subjekts mit mindestens einem visuellen Reiz, der zufällig aus einem Satz von visuellen Reizen, bestehend aus vorhalbierten Linien oder rechteckigen Reizen, trapezförmigen Reizen oder dreieckigen Reizen mit Polaritätsumkehr in variabler Position, ausgewählt ist, und Erfassung von dem Subjekt einer Antworten, die aus einem Satz von Antworten auf visuelle Reize ausgewählt ist;
c) Unterwerfen des Subjekts mit mindestens einem akustischen Reiz, der zufällig aus einem Satz von sinusförmigen oder komplexen akustischen Reizen ausgewählt ist, die in der binauralen Präsentation miteinander identisch sind, mit Änderungen der ILD- und/oder ITD- und/oder IPD-Parametern und Erfassen von dem Subjekt einer Antwort, die aus einem Satz von Antworten auf akustische Reize ausgewählt ist;
d) Wiederholen der Schritte b) und c) in zufälliger Reihenfolge für eine festgelegte Anzahl von Malen;
e) Berechnen eines numerischen Wertes, der repräsentativ für das neurologische Phänomen der visuell-räumlichen und audio-räumlichen Aufmerksamkeits-Lateralisierung ist, auf der Grundlage von Antworten, die das Subjekt auf verschiedene visuelle und akustische Reize gibt.

**2.** Verfahren nach Anspruch 1, wobei der Satz von visuellen Reizen horizontale Linien enthält, die in der Mitte, rechts von der Mitte und links von der Mitte halbiert sind.

**3.** Verfahren nach Anspruch 1, wobei der Satz von visuellen Reizen vertikale Linien enthält, die in der Mitte, unter der Mitte und über der Mitte halbiert sind.

**4.** Verfahren nach Anspruch 1, wobei der Satz von visuellen Reizen in Schritt b) von Anspruch 1 rechteckige, trapezförmige und dreieckige Reize mit Polaritätsinversion in der Mitte, rechts von der Mitte und links von der Mitte umfasst.

**5.** Verfahren nach Anspruch 1, wobei der Satz von akustischen Reizen in Schritt c) von Anspruch 1 ausgeglichene einfache Töne, einfache rechts lateralisierte Töne und einfache links lateralisierte Töne umfasst.

**6.** Verfahren nach Anspruch 1, wobei der Satz von Antworten auf die visuellen Reize, die in Schritt b) von Anspruch 1 erhalten werden, die Bewertung der Mittenhalbierung von horizontalen oder vertikalen Linien und Reize mit Polaritätsinversion umfasst.

7. Verfahren nach Anspruch 1, wobei der Satz von Antworten auf die akustischen Reize die Bewertung ausgewogener einfacher Töne umfasst.

8. Verfahren nach Anspruch 1, wobei mindestens einem in Schritt c) übermittelten akustischen Reiz ein Distraktor-Reiz vorausgeht.

9. Verfahren nach Anspruch 1, wobei die gleiche Anzahl von visuellen Reizen jedem Subjekt für jedes der zwei Halbfelder übermittelt wird und die gleiche Anzahl von akustischen Reize für jedes der beiden Halbfelder übermittelt wird.

10. Verfahren nach Anspruch 1, wobei der Schritt e) das individuelle Berechnen der prozentualen Anteile richtiger Antworten für jedes der beiden visuellen Halbfelder und für jedes der beiden akustischen Halbfelder umfasst.

11. Computerprogrammprodukt, umfassend einen Datenträger, in dem ausführbare Zeilencodes, die in maschinenlesbarer Form realisiert sind, zur Durchführung eines Verfahrens nach den Ansprüchen 1 bis 10 gespeichert sind, wenn in einer programmierter Testvorrichtung ausgeführt, um eine Testreihe zu einem Subjekt zu übermitteln und die entsprechende Antwort von dem Subjekt zu erfassen, wobei die Testvorrichtung mindestens einen Computer, mindestens einen Monitor zum Anzeigen von Bildern, mindestens ein Audiowiedergabegerät und Mittel zum Auswählen der Testantworten umfasst.

## Revendications

1. Procédé d'évaluation de phénomènes neurologiques de latéralisation hémisphérique, le procédé comprenant les étapes de :

a) fourniture d'un appareil de test programmé pour soumettre un sujet à une série de tests et acquérir les réponses respectives provenant du sujet, l'appareil de test comprenant au moins un ordinateur, au moins un dispositif d'affichage pour afficher des images, au moins un dispositif de reproduction audio et un moyen pour sélectionner les réponses de test ;
b) soumission du sujet à au moins un stimulus visuel sélectionné de façon aléatoire à partir d'un ensemble de stimuli visuels faits de lignes pré-recoupées, ou de stimuli rectangulaires, de stimuli trapézoïdaux ou de stimuli triangulaires avec inversion de polarité, dans une position variable, et acquisition auprès du sujet d'une réponse sélectionnée parmi un ensemble de réponses à des stimuli visuels ;
c) soumission du sujet à l'au moins un stimulus auditif sélectionné de façon aléatoire parmi un ensemble de stimuli auditifs sinusoïdaux ou complexes, identiques les uns aux autres dans une présentation binaurale, avec des changements des paramètres ILD et/ou ITD et/ou IPD, et acquisition auprès du sujet d'une réponse sélectionnée parmi un ensemble de réponses aux stimuli auditifs ;
d) répétition des étapes b) et c) dans un ordre aléatoire pour un nombre fixé de fois ;
e) calcul d'une valeur numérique représentative du phénomène neurologique de latéralisation d'attention visio-spatiale et audio-spatiale sur la base de réponses données par le sujet à différents stimuli visuels et auditifs.

2. Procédé selon la revendication 1, dans lequel l'ensemble desdits stimuli visuels inclut des lignes horizontales recoupées au centre, à droite du centre et à gauche du centre.

3. Procédé selon la revendication 1, dans lequel l'ensemble desdits stimuli visuels inclut des lignes verticales recoupées au centre, au-dessous du centre et au-dessus du centre.

4. Procédé selon la revendication 1, dans lequel ledit ensemble desdits stimuli visuels utilisés dans l'étape b) de la revendication 1 inclut des stimuli rectangulaires, trapézoïdaux et triangulaires avec inversion de polarité dans le centre, à droite du centre et à gauche du centre.

5. Procédé selon la revendication 1, dans lequel ledit ensemble desdits stimuli auditifs utilisés dans l'étape c) de la revendication 1 inclut des tonalités simples équilibrées, des tonalités simples latéralisées à droite et des tonalités simples latéralisées à gauche.

6. Procédé selon la revendication 1, dans lequel ledit ensemble de réponses auxdits stimuli visuels obtenu dans l'étape b) de la revendication 1 inclut l'évaluation de recoupage au centre de lignes horizontales ou verticales et de stimuli avec inversion de polarité.

7. Procédé selon la revendication 1, dans lequel l'ensemble de réponses auxdits stimuli auditifs inclut l'évaluation de tonalités simples équilibrées.

8. Procédé selon la revendication 1, dans lequel un seul stimulus auditif soumis à ladite étape c) est précédé par un stimulus leurre.

9. Procédé selon la revendication 1, dans lequel le même nombre de stimuli visuels est soumis à chaque sujet par chacun des deux hémichamps et le même nombre de stimuli auditifs est soumis par chacun des deux hémichamps.

10. Procédé selon la revendication 1, dans lequel ladite étape e) inclut un calcul de façon individuelle des pourcentages de réponses correctes par chacun des deux hémichamps visuels et par chacun des deux hémichamps auditifs.

11. Produit formant programme informatique, comprenant un support de données sur lequel des codes de lignes exécutables, qui sont réalisés sous forme lisible par une machine, sont stockés pour exécuter un procédé selon les revendications 1 à 10, quand il s'exécute dans un appareil de test programmé pour soumettre un sujet à une série de tests et acquérir la réponse respective auprès du sujet, l'appareil de test comprenant au moins un ordinateur, au moins un dispositif d'affichage pour afficher des images, au moins un dispositif de reproduction audio, et un moyen pour sélectionner des réponses de test.

Fig. 1

Fig. 2A

Fig. 2B

Fig. 2C

Fig. 3A

Fig. 3B

Fig. 3C

Fig. 4A

Fig. 4B

Fig. 4C

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4556069 A

**Non-patent literature cited in the description**

- **PIZZAMIGLIO L. ; JUDICA A. ; RAZZANO C. ; ZOCCOLOTTI P.** Toward a comprehensive diagnosis of visual-spatial disorders in unilateral brain damaged patients. *Evaluacion Psicologica,* 1989, vol. 5, 199-218
- **BOWERS D. ; HEILMAN K. M.** Pseudoneglect. Effects of hemispace on a tactile line bisection task. *Neuropsychologia,* 1980, vol. 18, 491-498
- **JEWELL G. ; MCCOURT M. E.** Pseudoneglect: A review and meta-analysis of performance factors in line bisection tasks. *Neuropsychologia,* 2000, vol. 38, 93-110
- **MCCOURT M. E.** Performance consistency of normal observers in forced-choice tachistoscopic visual line bisection. *Neuropsychologia,* 2001, vol. 39 (10), 1065-1076
- **MCCOURT M. E. ; GARLINGHOUSE M.** Stimulus modulation of pseudoneglect: Influence of line geometry. *Neuropsychologia,* 2000, vol. 38 (4), 520-524
- **SUAVANSRI K. ; FALCHOOK A. D. ; WILLIAMSON J. B. ; HEILMAN K. M.** Right up there: Hemispatial and hand asymmetries of altitudinal pseudoneglect. *Brain and cognition,* 2012, vol. 79 (3), 216-220
- **RUECKERT L. ; DERAVANESIAN A. ; BABOORIAN D. ; LACALAMITA A. ; REPPLINGER M.** Pseudoneglect and the cross-over effect. *Neuropsychologia,* 2002, vol. 40 (2), 162-173
- **CHEN P. ; GOEDERT K. M. ; MURRAY E. ; KELLY K. ; AHMETI S. ; BARRETT A. M.** Spatial bias and right hemisphere function: Sex-specific changes with aging. *Journal of the International Neuropsychological Society,* 2011, vol. 17, 455
- **SOSA Y. ; CLARKE A. M. ; MCCOURT M. E.** Hemifield asymmetry in the potency of exogenous auditory and visual cues. *Vision research,* 2011, vol. 51 (11), 1207-1215
- **BELLIS, T. J. ; BILLIET, C. ; ROSS, J.** Hemispheric lateralization of bilaterally presented homologous visual and auditory stimuli in normal adults, normal children, and children with central auditory dysfunction. *Brain and cognition,* 2008, vol. 66 (3), 280-289
- **MOSCATELLI, A. ; MEZZETTI, M. ; LACQUANITI, F.** Modeling psychophysical data at the population-level: The generalized linear mixed model. *Journal of vision,* 2012, vol. 12 (11), 26